# EUROPÄISCHE PATENTSCHRIFT

(11) **EP 2 434 965 B1**
(45) Veröffentlichungstag und Bekanntmachung des Hinweises auf die Patenterteilung: **08.04.2015**
(21) Anmeldenummer: 10714327.3
(22) Anmeldetag: 22.04.2010
(51) Int. Cl.: A61B 17/32, A61B 17/3207, A61B 17/00, A61B 17/29

(54) **CHIRURGISCHES INSTRUMENT**
SURGICAL INSTRUMENT
INSTRUMENT CHIRURGICAL

(30) Priorität: 29.05.2009 DE 102009024242; 14.09.2009 DE 102009042491
(43) Veröffentlichungstag der Anmeldung: 04.04.2012
(73) Patentinhaber: Aesculap AG, 78532 Tuttlingen (DE)
(72) Erfinder: HERMANN, Reiner, 78567 Fridingen (DE); HEGEMANN, Olaf, 44139 Dortmund (DE); LUTZE, Theodor, 78582 Balgheim (DE)
(74) Vertreter: Hoeger, Stellrecht & Partner Patentanwälte
(86) Internationale Anmeldenummer: PCT/EP2010/055397
(87) Internationale Veröffentlichungsnummer: WO 2010/136274

(56) Entgegenhaltungen:
- EP-A1- 0 445 918
- WO-A2-2007/039875
- DE-A1-102004 046 539
- US-A1- 2005 096 694
- US-A1- 2007 021 737
- US-A1- 2007 219 539

## Beschreibung

Die Erfindung betrifft ein chirurgisches Instrument für minimalinvasive chirurgische Eingriffe mit einem an einem so genannten distalen Ende eines Schafts an ein Antriebselement gekoppelten Werkzeug. Solche Instrumente werden häufig als so genannte Shaver verwendet.

Chirurgische Instrumente dieser Art weisen einen proximalen sowie einen distalen Endabschnitt und einen sich dazwischen erstreckenden Mittelabschnitt auf. Ein lang gestreckter hohler äußerer Schaft, ein in dem äußeren Schaft drehbar gelagertes, häufig hohlzylindrisches Antriebselement sowie ein am distalen Endabschnitt des Instruments angeordnetes, mit dem Antriebselement gekoppeltes Schneid-, Abrasiv- oder Fräswerkzeug bilden die wesentlichen Bauteile dieser Instrumente.

Neben linear ausgebildeten Instrumenten sind auch chirurgische Instrumente bekannt, bei denen der distale Endbereich abgewinkelt oder gekröpft ist, um mit dem chirurgischen Instrument auch schwieriger zugängliche Arbeitspositionen erreichen zu können und um den Arbeitsbereich des Instruments generell zu vergrößern.

Es ist in diesem Zusammenhang bekannt, das Antriebselement zwischen dem proximalen und distalen Endabschnitt mit einem flexiblen Abschnitt auszustatten, der eine Mehrzahl an Ringsegmenten umfasst, welche in Axialrichtung jeweils einen ersten und einen zweiten Endbereich aufweisen, wobei der erste Endbereich zwei oder mehr in Axialrichtung abstehende Vorsprünge und der zweite Endbereich zwei oder mehr die Vorsprünge aufnehmende Rücksprünge umfasst und die Ringsegmente über die Vor- und Rücksprünge gelenkig ineinander greifen.

Chirurgische Instrumente dieser Art sind beispielsweise aus der EP 0 677 276 B1 bekannt, wobei der flexible Abschnitt erlaubt, das Drehmoment vom proximalen Endabschnitt des Antriebselements wirksam auf den distalen Endabschnitt und damit auf das dort angeschlossene Werkzeug zu übertragen.

Da der Shaver typischerweise zur Entfernung von Körpergewebe dient, wird der sich im Inneren des Schafts und des (hohlzylindrischen) Antriebselements vorhandene Kanal zum Absaugen der entfernten Gewebeteile verwendet.

Nachteilig bei diesen bekannten Instrumenten ist, dass die Herstellung, insbesondere die Montage, des flexiblen Abschnittes aufwändig ist, da der flexible Abschnitt aus einer Vielzahl von Ringsegmenten nur lose zusammengesetzt ist, so dass nicht nur bei der Herstellung, sondern auch bei einer Demontage des Antriebselements und dessen Entnahme aus dem hohlzylindrischen äußeren Schaft Ringsegmente verloren gehen können, insbesondere auch im OP.

Ein weiterer Shaver, der diesem Prinzip der Konstruktion des Antriebselements mit im flexiblen Abschnitt lose ineinander greifenden Ringsegmenten folgt, ist aus der DE 10 2004 046 539 A1 bekannt. Dieser Shaver offenbart den Gegenstand der Präambel des Anspruchs 1.

Ein anderer Ansatzpunkt wurde in der EP 0 986 989 B1 gewählt, bei dem der flexible Abschnitt durch ein hohlzylindrisches Element gebildet wird, dessen Wandung schraubenlinienförmig in Radialrichtung geschlitzt ist, wobei sich mäanderförmig abwechselnd Zähne und Einbuchtungen entlang der Schraubenlinie abwechseln und ineinander greifen, so dass in Axialrichtung ein Zusammenhalt der Windungen gegeben ist.

Nachteilig bei dieser Lösung ist eine erheblich geringere Flexibilität und häufig vorkommende Ermüdungsbrüche, die durch die Wechselbiegebeanspruchung beim Drehen der Welle auftreten. Ein oszillierender Antrieb soll mit diesem Instrument zwar möglich sein, jedoch ist es mit hohen Drehzahlen nur in einer Drehrichtung antreibbar.

Aus der US 2007/0021737 A1 ist ein chirurgisches Instrument bekannt, dessen distales Ende gegenüber einem Mittelabschnitt verschwenkbar ist. Hierzu ist am proximalen Ende verschwenkbar ein Handgerät angeordnet, mit dem auch das zangenartige Werkzeug betätigbar ist.

Aufgabe der Erfindung ist es, ein chirurgisches Instrument der eingangs beschriebenen Art so weiterzubilden, dass bei minimalem Aufwand in der Herstellung ein sicherer Betrieb, insbesondere auch bei hohen Drehzahlen, möglich ist. Ein weiterer Aspekt liegt in der Bereitstellung eines Instruments, das in einer Vielzahl unterschiedlicher Arbeitssituationen einsetzbar ist.

Diese Aufgabe wird erfindungsgemäß bei einem chirurgischen Instrument dadurch gelöst, dass die Ringsegmente über die Vor- und Rücksprünge in Axial- und Radialrichtung miteinander formschlüssig verbunden sind, und dass der äußere Schaft eine im Bereich des distalen Endabschnitts des Instruments angeordnete Gelenkzone umfasst, die den distalen Endabschnitt und den Mittelabschnitt des Instruments gelenkig miteinander verbindet, wobei der flexible Abschnitt des Antriebselements eine Länge in Axialrichtung aufweist, die zumindest im Wesentlichen der Länge der Gelenkzone des äußeren Schafts in Axialrichtung entspricht.

Durch den Formschluss der Vor- und Rücksprünge in Axial- und Radialrichtung, wird die Handhabung des Antriebselements bei der Herstellung, beim Zusammenbau sowie bei einer späteren Demontage erheblich erleichtert. Die gelenkige Verbindung der Ringsegmente im flexiblen Abschnitt ist trotzdem gewährleistet..

Werden die Ringsegmente mit mehr als zwei Vor- und Rücksprüngen versehen, lassen sich pro Segmentpaar zwei oder mehr Gelenkachsen realisieren.

Aufgrund der Ausbildung einer Gelenkzone im distalen Endabschnitt ist dieser mit dem Mittelabschnitt gelenkig verbunden. Das Instrument kann wahlweise in gerader Ausrichtung von Mittelabschnitt und distalem Endabschnitt betrieben werden oder auch in einer Vielzahl an mehr oder weniger stark gekröpften Konfigurationen, die vom Operateur einstellbar und insbesondere auch während des Arbeitens mit dem Instrument veränderbar sind.

Der Formschluss der Ringsegmente in Axial- und Radialrichtung vermindert in erheblichem Umfang das Risiko einzelne Ringsegmente zu verlieren

Über den Formschluss in Axial- und Radialrichtung kann auch die gegenseitige Positionierung der aneinandergrenzenden Ringsegmente so genau vorgegeben werden, dass hohe Drehzahlen auch in beiden möglichen Antriebsrichtungen realisiert werden können.

Der Formschluss in Axialrichtung lässt sich durch die Verwendung von mehr als zwei Vor- und Rücksprüngen verbessern.

Typischerweise werden die Vor- und Rücksprünge in regelmäßigen Abständen in Umfangsrichtung am jeweiligen Ringsegment angeordnet. Dies gewährleistet eine gleichmäßige Belastung der Ringsegmente bei der Übertragung der Antriebskräfte und damit eine möglichst große Lebensdauer.

Gemäß einer Ausführungsform der vorliegenden Erfindung ist die Zahl der Vorsprünge und Rücksprünge ungerade gewählt, woraus sich insbesondere bei einer Anordnung der Vor- und Rücksprünge in regelmäßigen Abständen in Umfangsrichtung am jeweiligen Ringsegment ein Formschluss in Radialrichtung ohne weitere Maßnahmen ergibt.

Zusätzlich oder alternativ kann der Formschluss in Radialrichtung dadurch erreicht werden, dass die Vorsprünge an ihrer radial außen liegenden Seite in Umfangsrichtung eine größere Ausdehnung aufweisen als auf ihrer radial innen liegenden Seite und dass die Rücksprünge entsprechend auf ihrer radial innen liegenden Seite in Umfangsrichtung eine Ausdehnung aufweisen, die kleiner ist als die entsprechende radial außen liegende Ausdehnung im entsprechenden Bereich des in den Rücksprung eingreifenden Vorsprungs.

Mit dieser Maßnahme kann ein Formschluss in Radialrichtung erzielt werden, unabhängig davon, ob die Zahl der Vor- und Rücksprünge ungerade oder gerade ist.

Der Formschluss in Axialrichtung kann dadurch erreicht werden, dass die Vorsprünge an ihrem dem Ringsegment abgewandten, freien Ende eine größere Ausdehnung in Umfangsrichtung aufweisen als an ihrem zum Ringsegment benachbarten Ende und die Rücksprünge an ihrem offenen Ende eine kleinere Ausdehnung in Umfangsrichtung aufweisen als die Ausdehnung des in den Rücksprung eingreifenden Vorsprungs an seinem freien Ende in Umfangsrichtung.

Wird ein Formschluss sowohl in Axialrichtung als auch in Radialrichtung verwirklicht, hat man ein Antriebselement vorliegen, das als Ganzes gehandhabt werden kann ohne die Gefahr, dass einzelne Teile des flexiblen Abschnitts verloren gehen.

Vorzugsweise wird ein solches Antriebselement mit einem flexiblen Abschnitt so hergestellt, dass zunächst ein einstückiges, hohlzylindrisches Element verwendet wird, bei dem beispielsweise mit Laser die Konturen der Vor- und Rücksprünge eingeschnitten werden. Häufig reicht der dabei entstehende Schneidspalt bereits aus, um eine ausreichende Verschwenkung der einzelnen Ringsegmente gegeneinander sicherzustellen, so dass der flexible Abschnitt des Antriebselements einen für die jeweilige Anwendung ausreichenden Biegewinkel bereitstellen kann.

Ist in Axialrichtung bereits ein Formschluss vorhanden, so kann der Formschluss in Radialrichtung auch dadurch erzielt werden, dass die Vorsprünge in Axialrichtung auf der radial außen liegenden Seite eine größere Ausdehnung in Längsrichtung aufweisen als auf der radial innen liegenden Seite und die Rücksprünge auf der radial innen liegenden Seite eine Tiefe in Axialrichtung aufweisen, die geringer ist als die Länge der Vorsprünge in Axialrichtung auf deren außen liegenden Seite.

Bei einer bevorzugten Ausführungsform der Erfindung werden in Umfangsrichtung Anlageflächen auf Seiten der Vorsprünge als auch korrespondierend bei den Rücksprüngen vorgesehen, die eben sind. Damit lässt sich die Drehmomentübertragung optimieren, woraus ein geringerer Verschleiß und die Möglichkeit, sehr hohe Drehzahlen zuzulassen, resultieren.

Eine bevorzugte Form der Vorsprünge in Umfangsrichtung ist die Trapezform, auch wenn selbstverständlich hiervon abweichende Formen die Realisierung eines Formschlusses in Axialrichtung ermöglichen. Korrespondierend hierzu sind die Rücksprünge in Umfangsrichtung vorzugsweise trapezförmig ausgebildet. Hier ergeben sich dann großflächige ebene Anlageflächen der Vor- und Rücksprünge.

Für die Sicherstellung eines Formschlusses in Radialrichtung sind die Vorsprünge vorzugsweise mit einem in der Radialrichtung gesehenen trapezförmigen Querschnitt ausgebildet, wobei hier die Wölbungen an der radial außen liegenden und radial innen liegenden Oberfläche der Vorsprünge bei dieser Definition unberücksichtigt bleiben.

Korrespondierend hierzu weisen die Rücksprünge in Radialrichtung ebenfalls vorzugsweise einen trapezförmigen Querschnitt auf.

Wie weiter oben schon angesprochen, besteht ein Aspekt der Erfindung darin, das eingangs genannte chirurgische Instrument so weiter zu bilden, dass es flexibler einsetzbar ist und einen größeren Arbeitsbereich aufweist. Dies gelingt mit der im distalen Endabschnitt vorgesehenen Gelenkzone.

Die Einstellung des Anstellwinkels des distalen Endabschnitts gegenüber dem Mittelabschnitt geschieht bevorzugt mit einem Steuerungselement. Die dabei verursachte Auslenkung des distalen Endabschnitts ist dabei vorzugsweise reversibel.

Das erfindungsgemäße chirurgische Instrument weist bevorzugt ein Steuerungselement mit zwei oder mehr sich zumindest im Wesentlichen vom proximalen zum distalen Endabschnitt des Instruments erstreckenden, Zug- und/oder Druckkräfte übertragenden Längselementen auf. Die Längselemente sind dabei in Umfangsrichtung des Instruments in im Wesentlichen regelmäßigen Winkelabständen angeordnet.

Weiter bevorzugt sind die Kraft übertragenden Längselemente an ihrem proximalen und distalen Ende miteinander in Umfangsrichtung fest verbunden. Der proximale Endabschnitt weist bei einer solchen Ausführungsform bevorzugt ebenfalls eine Gelenkzone auf.

Aufgrund dieser Ausgestaltung des chirurgischen Instrumentes lassen sich nun am proximalen Endabschnitt Schwenkbewegungen durchführen, zu denen dann Schwenkbewegungen am distalen Endabschnitt korrespondieren. Die Kopplung der Schwenkbewegungen am proximalen und distalen Endabschnitt wird durch das Steuerungselement und dessen Kraft übertragende Längselemente erreicht.

Gegenüber den Instrumenten des Standes der Technik ist anstelle der geradlinigen oder feststehend gekröpften Konfiguration eine bedarfsweise geradlinig oder einstellbar gekröpfte Konfiguration möglich, die auch noch während eines chirurgischen Einsatzes bei einer Operation in vorgegebenen Grenzen variiert werden kann.

Werden zwei Kraft übertragende Längselemente verwendet, ist die Schwenkbewegung auf eine Ebene beschränkt. Werden mehrere, insbesondere vier oder mehr, beispielsweise acht Kraft übertragende Längselemente verwendet, besteht die Möglichkeit, das chirurgische Instrument in zwei aufeinander senkrecht stehenden Ebenen zu verschwenken oder aber, insbesondere bei der Verwendung von acht Steuerungselementen oder mehr in praktisch beliebig wählbaren Ebenen zu verschwenken.

Die Schwenkbewegungen sind dabei nicht auf Winkel von ca. 20° beschränkt, sondern diese können durchaus Werte bis weit über 90° erreichen.

Bei einer bevorzugten Ausführungsform der Erfindung weist das Instrument ein Steuerungselement auf, welches ein hohlzylindrisches Bauteil umfasst, dessen Zylinderwand mindestens im Bereich eines Abschnitts zwischen dem proximalen und distalen Ende in zwei oder mehr Wandsegmente unterteilt ist, die die Kraft übertragenden Längselemente bilden.

Hierbei können die zwei oder mehr Wandsegmente am distalen Ende des hohlzylindrischen Bauteils über einen Ringbund fest miteinander verbunden sein.

Weiterhin können die zwei oder mehr Wandsegmente im Bereich des proximalen Endes des hohlzylindrischen Bauteils fest miteinander verbunden sein.

Besonders bevorzugt wird das hohlzylindrische Bauteil einstückig ausgebildet. Hier ist die Handhabung beim Zusammenbau des Instruments besonders einfach. Außerdem lässt sich das einstückige Bauteil mit besonderer Präzision bezüglich der gegenseitigen Ausrichtung der Wandsegmente herstellen.

Instrumente dieser Ausgestaltung weisen insbesondere ein hohlzylindrisches Bauteil auf, welches aus einem einzigen Röhrchen gefertigt ist, wobei die Unterteilung der Zylinderwand in Wandsegmente vorzugsweise mittels Laserstrahlschneiden erfolgt.

Als Werkstoff zur Herstellung des Steuerungselements, insbesondere des hohlzylindrischen Bauteils bieten sich insbesondere Stahllegierungen oder Nitinol an.

Bei einer besonders bevorzugten Ausführungsform der Erfindung wird ferner ein innerer hohlzylindrischer Schaft verwendet, der als Antriebselement des Instruments ausgebildet werden kann. So bleibt ein möglichst großes inneres Lumen frei, beispielsweise zum Abführen von von dem Werkzeug abgetragenen Gewebeteilen des behandelten Patienten.

Das Antriebselement verfügt in einer solchen bevorzugten Ausführungsform über zwei flexible Abschnitte, die im zusammengebauten Zustand des Instruments jeweils in den proximalen und distalen Gelenkzonen innerhalb des äußeren Schafts angeordnet sind. Damit wird erreicht, dass die typischerweise rotierende Antriebsbewegung auch im abgewinkelten Zustand auf das an den distalen Endabschnitt angeschlossene Werkzeug übertragen werden kann.

Neben den eingangs genannten Schneid-, Abrasiv- und Fräswerkzeugen können auch Bohrwerkzeuge zur Verwendung kommen, wobei dann das Instrument anstelle einer seitlichen Öffnung an seinem distalen Ende eine Öffnung in Axialrichtung zum Durchtritt des Bohrwerkzeugs aufweist.

Um hier eine möglichst effektive Übertragung der Drehmomente zu erreichen, wird das Antriebselement im Wesentlichen verwindungssteif ausgebildet.

Auch der äußere Schaft ist vorzugsweise verwindungssteif ausgebildet um die beim Betrieb des Werkzeugs auftretenden Reaktionskräfte aufzunehmen und eine Verwindung des Instruments zu vermeiden. Eine Verwindung des Instruments hätte zur Folge, dass das Instrument von seinem jeweils vorgesehen Einsatzort wegbewegt wird, ein Effekt, der bei hochpräzise auszuführenden Operationen zu erheblichen Komplikationen führen könnte.

Bei einer weiteren Ausführungsform der vorliegenden Erfindung werden die Gelenkzonen biegeelastisch ausgebildet, so dass bei dem chirurgischen Instrument bei einem Fortfall der Kräfte, die eine Schwenkbewegung am proximalen Ende erzwingen, eine Rückstellung in die gerade Form bewirkt wird.

Die Kraft übertragenden Längselemente sind bei einer Variante der vorliegenden Erfindung zueinander lateral beabstandet angeordnet, so dass diese bei der Schwenkbewegung nicht aneinander reiben und so die Schwenkbewegung mit einem minimalen Kraftaufwand vorgenommen werden kann.

Alternativ kann zwischen den lateral beabstandeten Längselementen jeweils ein Abstandshalter angeordnet sein, so dass die Position in Umfangsrichtung der Längselemente auch bei größeren einzuleitenden Kräften zur Durchführung der Schwenkbewegung im Wesentlichen unverändert bleibt.

Alternativ kann vorgesehen sein, dass die Kraft übertragenden Längselemente entlang der Längsrichtung mindestens partiell in direktem Kontakt miteinander angeordnet sind. Auch hier ist sichergestellt, dass die Längselemente in Umfangsrichtung gesehen auch bei Krafteinleitung in ihren Positionen verbleiben und somit eine exakte Steuerung der Schwenkbewegung des distalen Endes erzielt werden kann.

Weiter bevorzugt werden die Kraft übertragenden Längselemente von dem äußeren und dem inneren Schaft in Radialrichtung geführt, was zu einer weiteren Verbesserung der Genauigkeit der am distalen Ende ausgeführten Schwenkbewegung führt.

Gemäß einer weiteren Ausführungsform der vorliegenden Erfindung kann vorgesehen sein, dass bei dem erfindungsgemäß einzusetzenden Steuerungselement die distalen Enden der Längselemente in Umfangsrichtung in Winkelpositionen festgelegt sind, die von den Winkelpositionen, in denen die jeweils zugehörenden proximalen Enden festgelegt sind, verschieden sind.

Dies erlaubt die Durchführung von Schwenkbewegungen des distalen Endes in einer anderen Ebene als der, in der die Schwenkbewegung des proximalen Endes durchgeführt wird.

Die Winkeldifferenz, in der die Winkelpositionen des distalen und des proximalen Endes eines Längselements festgelegt sind, kann im Bereich von ca. 10° bis ca. 350° reichen. Insbesondere sind Differenzen in den Winkelpositionen am proximalen und distalen Ende im Bereich von ca. 45° bis ca. 315° von Interesse, weiter bevorzugt im Bereich von ca. 150° bis ca. 210°.

Um dies zu erreichen, werden die Kraft übertragenden Längselemente vorzugsweise mindestens abschnittsweise schraubenlinienförmig angeordnet.

Im Hinblick auf die typische Länge eines chirurgischen Instruments und der daraus folgenden Länge der Längselemente und des gleichzeitig relativ geringen Durchmessers ergeben sich Winkelanstellungen der Längselemente auf ihrem schraubenlinienförmigen Weg, die in sehr geringem Umfang von der Axialrichtung des Instruments abweichen. Dies bedeutet, dass auch bei einem sehr großen Winkelversatz von beispielsweise 180° eine sichere Handhabung des Instruments gewährleistet ist und insbesondere auch die Schwenkbewegung des distalen Endes winkelgenau und vorhersagbar durchgeführt werden kann.

Bei einer weiteren Ausführungsform der Erfindung kann vorgesehen sein, dass die Kraft übertragenden Längselemente im Bereich des proximalen und/oder distalen Endabschnitts mit einer im Wesentlichen parallelen Ausrichtung zur Längsachse des Instruments angeordnet sind.

Alternativ können auch ein oder mehrere Abschnitte parallel zur Längsrichtung des Instruments angeordnet sein.

Auch hier ergibt sich im Hinblick auf die typische Länge des erforderlichen Steuerungselements von zumeist mehr als 10 cm und bei einem typischen Durchmesser des Instruments von wenigen Millimetern eine extrem hohe Steigung der Schraubenlinienform oder anders ausgedrückt eine sehr geringe Abweichung von der Parallelität zur Längsrichtung des Instruments, die wenige Winkelgrade bis zu einem Bruchteil eines Winkelgrads beträgt.

Gemäß einer Variante des erfindungsgemäßen Instruments sind die Kraft übertragenden Längselemente als Kabel oder Drähte ausgebildet.

Bei einer anderen Variante weisen die Kraft übertragenden Längselemente einen bananenförmigen Querschnitt auf.

Wie zuvor schon ausgeführt, werden bei einer besonders bevorzugten Ausführungsform die Kraft übertragenden Längselemente aus einem hohlzylindrischen Bauteil gebildet, bei dem, beispielsweise mittels Laserstrahlschneiden, die Zylinderwand über den größten Teil, insbesondere nahezu über die gesamte Länge in Axialrichtung zur Ausbildung der Kraft übertragenden Längsele-mente geschlitzt ist. Die Längselemente werden dabei von Zylinderwand-segmenten gebildet, die im Querschnitt eine Kreisbogenform aufweisen.

Bevorzugt weisen die Wandsegmente im Querschnitt eine Kreisbogenform auf, die einem Bogenwinkel von ca. 20° oder mehr, insbesondere 30° oder mehr entspricht.

Die Zahl der Wandsegmente liegt bevorzugt im Bereich von 4 bis 16, weiter bevorzugt im Bereich von 6 bis 12.

Der Abstand der Wandsegmente in Umfangsrichtung von einander (entspricht der Schlitzbreite) beträgt in Winkelgraden gemessen vorzugsweise ca. 2° bis 15°, weiter bevorzugt ca. 4° bis ca. 8°.

Die Schlitzbreite, wie sie beim Laserstrahlschneiden entsteht, kann bei Bedarf vergrößert werden, so dass die verbleibenden streifenförmigen Wandsegmente berührungslos gegeneinander bewegt werden können. Aufgrund der kreissegmentartigen Querschnitte der Längselemente bleibt der berührungslose Zustand der Längselemente auch im Falle der Zug- oder Druckbelastung auch in den Gelenkbereichen erhalten; dies gilt insbesondere bei einer Führung der Längselemente in Radialrichtung zwischen einem inneren und einem äußeren Schaft.

Die beiden Endbereiche des hohlzylindrischen Elements bleiben ungeschlitzt, so dass die Längselemente über Ringbünde miteinander verbunden bleiben.

Die proximalen und distalen Gelenkzonen des Instruments können in verschiedener Weise realisiert werden.

Wird der innere Schaft als Antriebselement verwendet, besitzt er von vornherein im Bereich der Gelenkzonen flexible Abschnitte, die zur Realisierung der proximalen und distalen Gelenkzonen ausreichend sein können. Dies bedeutet, dass der äußere Schaft in den Endabschnitten entsprechend flexibel sein muss, um den durch das Steuerungselement initiierten Schwenkbewegungen ebenfalls zu folgen.

Die Biegefestigkeit im Mittelabschnitt kann durch eine biegefeste Ausgestaltung des inneren und/oder äußeren Schafts gewährleistet werden.

Alternativ kann sowohl der innere als auch der äußere Schaft im Bereich der proximalen und distalen Gelenkzonen einen proximalen und distalen Gelenkabschnitt aufweisen, wobei bei der Verwendung des inneren Schafts als Antriebselement dessen flexible Abschnitte dem proximalen bzw. distalen Gelenkabschnitt entsprechen.

Vorzugsweise weisen die Gelenkzonen des äußeren und/oder inneren Schafts in Umfangsrichtung verlaufend mehrere Schlitze auf, die voneinander durch Wandbereiche in Umfangsrichtung bzw. Axialrichtung voneinander getrennt sind.

Bevorzugt weist ein jeweiliger Wandabschnitt in Umfangsrichtung zwei oder mehr, insbesondere drei oder mehr Schlitze hintereinander angeordnet auf. Die Schlitze sind dabei bevorzugt in Umfangsrichtung mit gleichen Abständen zueinander angeordnet.

In Axialrichtung weisen die Gelenkzonen bevorzugter Instrumente drei oder mehr Schlitze nebeneinander angeordnet auf, wobei bevorzugt die nebeneinander angeordneten Schlitze in Umfangsrichtung gegeneinander versetzt angeordnet sind. Die Abstände, in denen die Schlitze in Axialrichtung zu einander beabstandet angeordnet sind, können gleich sein oder variieren, wobei hiermit die Gelenkeigenschaften, insbesondere der Biegeradius, beeinflusst werden können.

Typischerweise wird vorgesehen, dass die Schlitze die Zylinderwand vollständig durchdringende Schlitze sind. Gute Biegeeigenschaften lassen sich allerdings auch erzielen, wenn die Schlitze die Wand des Schafts nicht vollständig durchsetzen, sondern insbesondere vor dem Erreichen des Innenumfangs enden. Damit bleibt die Wand des Schafts insgesamt geschlossen, was in einigen Anwendungen insbesondere beim äußeren Schaft erwünscht sein kann.

Eine bevorzugte Geometrie der Schlitze liegt vor, wenn die die Schlitze begrenzenden Wandflächen in einem spitzen Winkel zur Radialrichtung angeordnet sind. Vorzugsweise werden dabei gegenüberliegende Wandflächen desselben Schlitzes spiegelbildlich angeordnet, so dass sich am Außenumfang eines Schafts eine größere Schlitzbreite ergibt als benachbart zum Innenumfang.

In Axialrichtung von einander beabstandete Schlitze werden vorzugsweise in Umfangsrichtung überlappend, jedoch gegeneinander versetzt angeordnet, so dass sich eine regelmäßige Anordnung der Schlitze ergibt.

Die Wandflächen der Schlitze können dabei gegen die Axialrichtung unter einem Winkel geneigt sein, der von 90 ° abweicht, so dass die Breite der Schlitze am Außenumfang größer ist als am Innenumfang des äußeren Schafts. Damit lassen sich auch bei kleinen Schlitzbreiten ausreichend große Schwenkwinkel realisieren, ohne dass die Zahl der Schlitze vergrößert werden müsste bzw. der Gelenkbereich sich über eine größere axiale Länge erstrecken müsste.

Während in vielen Fällen die proximale und die distale Gelenkzone gleich ausgebildet sind und insbesondere eine gleiche Ausdehnung in Längsrichtung des Instruments aufweisen, ist dies nicht zwingend erforderlich.

Insbesondere kann vorgesehen sein, dass die proximale und die distale Gelenkzone verschieden, insbesondere auch verschieden lang ausgebildet sind. Dadurch lässt sich beispielsweise erreichen, dass eine entsprechende Schwenkbewegung der proximalen Gelenkzone in einer geringeren oder verstärkten Schwenkbewegung am distalen Endabschnitts des Instruments resultiert.

Insbesondere kann vorgesehen sein, dass die Schwenkbewegung der proximalen und/oder distalen Gelenkzone einstellbar ist. Dies kann beispielsweise dadurch geschehen, indem die Ausdehnung der proximalen und/oder der distalen Gelenkzone variiert wird und damit das Schwenkverhalten der beiden Gelenkzonen zueinander verändert wird.

Insbesondere kann vorgesehen sein, dass das Instrument eine Haltevorrichtung umfasst, mit der Teile einer der Gelenkzonen biegefest bezüglich des Mittelabschnitts oder eines sich an den proximalen oder distalen Endabschnitt des Instruments anschließenden Funktionseinheit fixierbar sind.

So kann bei einer Variante des erfindungsgemäßen Instruments die Haltevorrichtung eine parallel zur Längsachse des biegefesten Mittelabschnitts eine verschiebliche, biegesteife Hülse umfassen. Je nach Position der Hülse in Längsrichtung zum Mittelabschnitt kann der proximale und/oder distale Endabschnitt und die dort vorgesehene Gelenkzone in ihrer Länge beeinflusst und damit in ihrem Schwenkverhalten ebenfalls beeinflusst werden.

Vorzugsweise wird dabei die biegesteife Hülse am Außenumfang des biegesteifen Schafts angeordnet, so dass nicht nur das Lumen der Steuerungsvorrichtung unbeeinflusst bleibt, sondern auch die Position der Hülse einfach veränderlich und insbesondere auch festlegbar ist.

Gemäß einer anderen Variante kann die Haltevorrichtung an der Funktionseinheit, die an das proximale Ende der Steuerungsvorrichtung gekoppelt ist, ein abstützendes Halteelement umfassen. Auf diese Weise lässt sich die Gelenkzone vonseiten des proximalen Endes her in ihrem Schwenkverhalten beeinflussen.

Gemäß einer weiteren Variante des erfindungsgemäßen Instruments ist die Haltevorrichtung in einer vorgegebenen Stellung positionierbar und insbesondere auch festlegbar. Damit besteht die Möglichkeit, wiederholbar und genau vorgebbar das Schwenkverhalten von distalem und proximalem Endabschnitt zueinander vorab einzustellen oder wieder einzustellen.

Diese und weitere Vorteile der Erfindung werden im Folgenden anhand der Zeichnung noch näher erläutert. Es zeigen im Einzelnen:
- Figur 1A: eine perspektivische Darstellung eines erfindungsgemäßen chirurgischen Instruments in Form eines Shavers;
- Figur 1B: eine Schnittdarstellung des Shavers von Figur 1A;
- Figur 2: eine weitere Ausführungsform des erfindungsgemäßen Instruments;
- Figur 3A, B und C: einen äußeren Schaft, ein Steuerungselement sowie einen inneren Schaft des erfindungsgemäßen Instruments gemäß Figur 2;
- Figur 3D: eine alternative Ausführungsform des Steuerungselements von Figur 3B;
- Figur 3E und F: zwei alternative Ausführungsformen von Gelenkabschnitten für den äußeren Schaft der Figur 3A;
- Figur 4 A und B: alternative Ausführungsformen eines Steuerungselements für das erfindungsgemäße chirurgische Instrument der Figur 3B;
- Figur 5: eine Einzelheit eines flexiblen Antriebselements des Instruments der Figur 1B;
- Figur 6: eine schematische Darstellung einer ersten Weiterbildung des erfindungsgemäßen Instruments von Figur 2; und
- Figur 7A und B: eine schematische Darstellung einer zweiten Weiterbildung des erfindungsgemäßen chirurgischen Instruments von Figur 2.

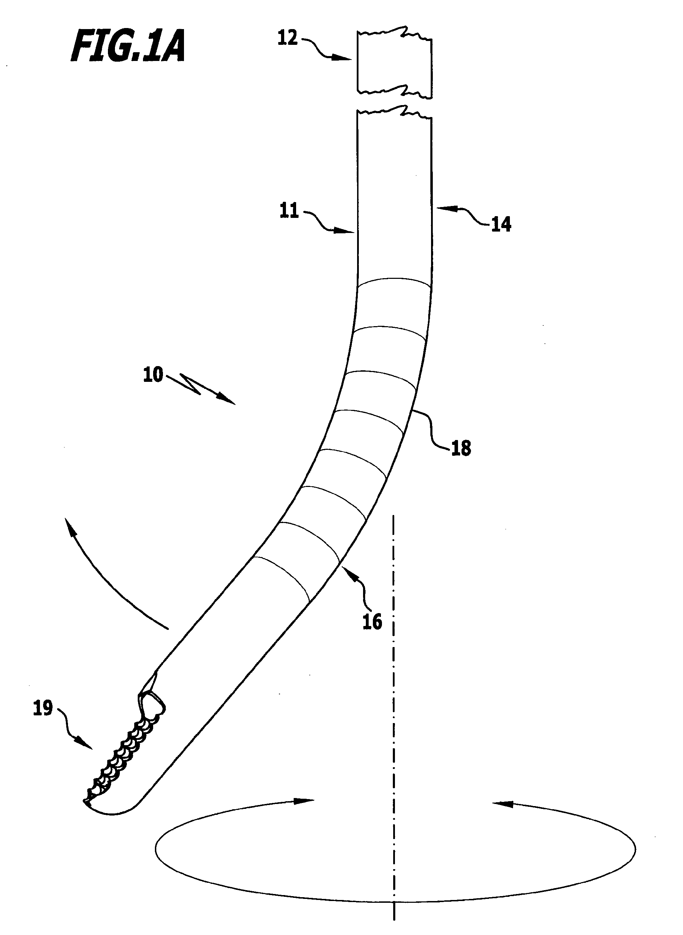

Figur 1A zeigt ein erfindungsgemäßes chirurgisches Instrument in Form eines Shavers 10 mit einem mit einem hohlzylindrischen äußeren Schaft 11, der sich in einen proximalen Endabschnitt 12, einen geraden, biegesteifen Mittelabschnitt 14 sowie einen leicht abgewinkelten distalen Endabschnitt 16 mit einer Gelenkzone 18 gliedert, an den ein Werkzeug 19, beispielsweise ein Bohr-, Schneid-, Abrasiv- oder Fräswerkzeug angeschlossen, insbesondere auch angeformt ist.

Der Winkel, mit dem der distale Endabschnitt 16 von der Längsrichtung des Mittelabschnitts 14 des Instruments 10 abweicht, ist aufgrund der Gelenkzone 18 variabel. Dies erleichtert das Arbeiten in schwierig zugänglichen Arbeitspositionen erheblich.

Das Instrument kann durch Drehung um die Längsachse des Mittelabschnitts 14, der typischerweise in einem Trokar geführt wird, einen zusätzlichen Arbeitsbereich abdecken, der gegenüber der vorbekannten, geradlinigen Ausführung des Instruments deutlich erweitert ist.

In dem hohlzylindrischen Schaft 11 ist ein drehbar gelagertes, vorzugsweise hohlzylindrisches Antriebselement 20 aufgenommen.

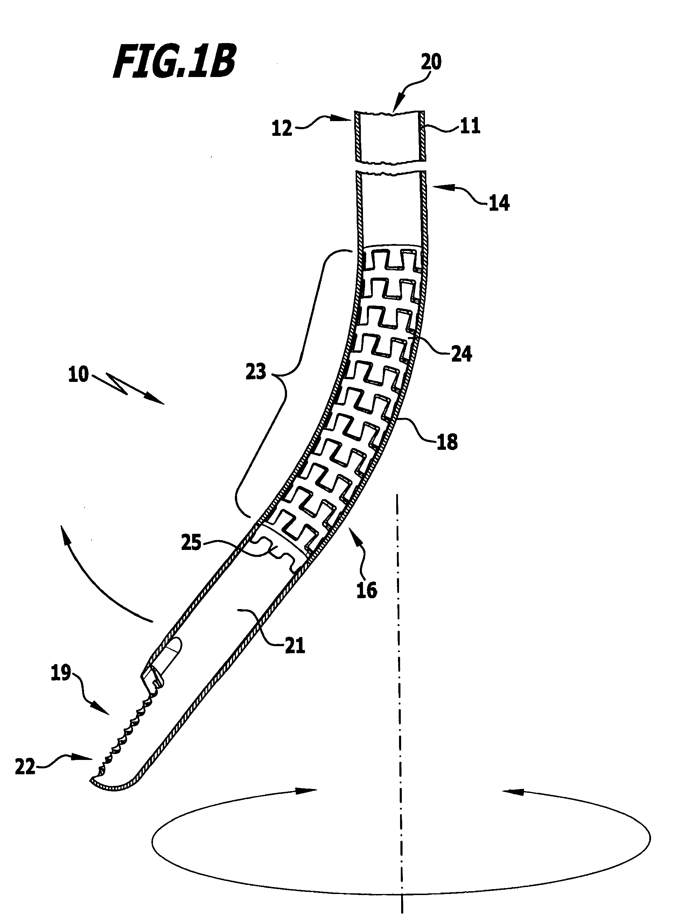

An das Antriebselement 20 ist an seinem distalen Ende 21 ein Werkzeug 22 angekoppelt, das ebenso wie der äußere Schaft 11 eine seitliche Öffnung aufweist. Die Öffnungen sind, wie aus Figur 1B ersichtlich, an ihren Rändern angeschliffen und mit Schneiden versehen. Wird das Antriebselement 20 in Rotation versetzt, so werden in den Bereich der seitlichen Öffnungen gelangende Gewebeteile durch die Schneiden der Ränder der Öffnungen abgetrennt und können über den im hohlzylindrischen Antriebelement 20 verbleibenden Innenraum mittels Unterdruck abgesaugt werden.

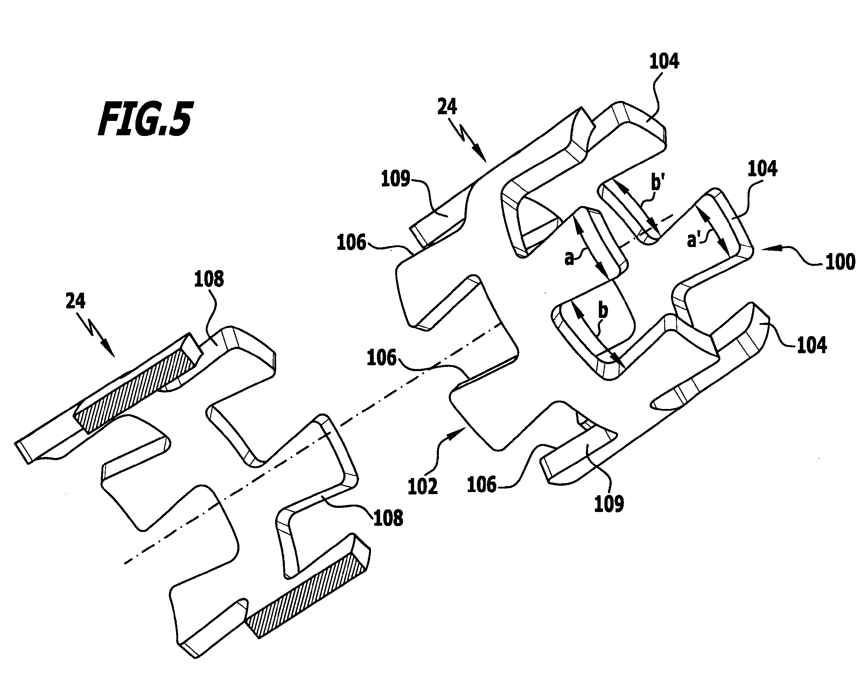

Im distalen Endabschnitt 16 des Shavers 10, d.h. im Bereich der Gelenkzone 18, geht das hohlzylindrische Antriebselement 20 aus einem einstückigen oder massiven hohlzylindrischen Teil in einen hohlzylindrischen flexiblen Abschnitt 23 über, der von einer Anzahl an Ringsegmenten 24 gebildet wird, die im Folgenden anhand der Figur 5 noch näher beschrieben werden.

Das distale Ende des Antriebselements 20 endet typischerweise mit einem Kupplungsstück 25, an das lose eingreifend ein Werkzeug, insbesondere das Fräswerkzeug 22, angekoppelt werden kann.

Das Fräswerkzeug 22 kann so einfach, beispielsweise nach Verschleiß, ausgetauscht werden und durch ein neues oder bei Bedarf durch anderes Werkzeug ersetzt werden. Alternativ kann das Werkzeug 22 fest und unverlierbar mit dem distalen Ende 21 des Antriebselements 20 verbunden werden.

Die Einstellung des Winkels, mit dem der distale Endabschnitt 16 gegen den Mittelabschnitt geneigt ist, kann mittels an sich bekannter Steuerungselemente, beispielsweise Bowdenzügen, eingestellt werden. Diese Einzelheit ist in Figur 1B nicht gezeigt.

Das distale Ende des Instruments 10 ist in den Figuren 1A und 1B jeweils geschlossen mit einer seitlichen Öffnung gezeigt. Verwendet man anstelle eines Fräswerkzeugs ein Bohrwerkzeug wird anstelle der seitlichen Öffnung eine Öffnung in Axialrichtung vorgesehen, die am distalen Ende in Axialrichtung das Bohrwerkzeug freigibt (nicht gezeigt).

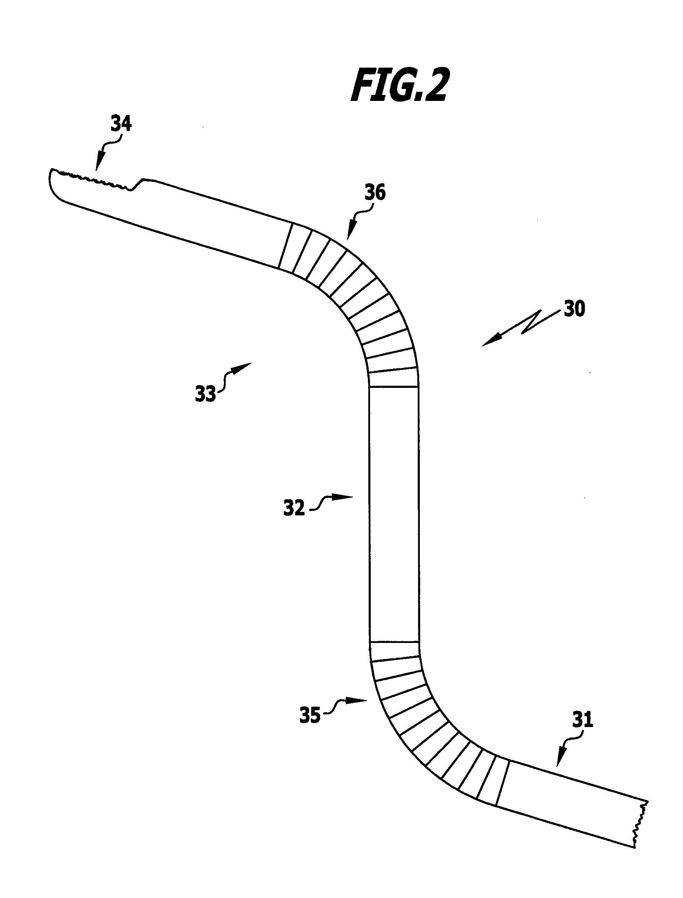

Gemäß der vorliegenden Erfindung wird ein chirurgisches Instrument bevorzugt sowohl mit einer proximalen als auch einer distalen Gelenkzone versehen, wie dies am Beispiel des Shavers 30 der Figur 2 gezeigt ist und im Folgenden diskutiert werden soll.

Der erfindungsgemäße Shaver 30 weist einen Schaft auf, der sich in einen proximalen Endabschnitt 31, einen biegesteifen Mittelabschnitt 32 sowie einen distalen Endabschnitt 33 gliedert.

An den distalen Endabschnitt 33 ist ein Werkzeug 34 angeschlossen oder angeformt, das in seiner Ausbildung dem beispielsweise in der DE 10 2004 046 539 A1 beschriebenen entsprechen kann.

Der proximale und der distale Endabschnitt 31, 33 des Instruments 30 umfassen jeweils eine Gelenkzone 35, 36, die eine Schwenkbewegung des proximalen Endabschnitts 31 erlauben, die sich aufgrund eines Steuerungselements des Instruments 30 in eine Schwenkbewegung des distalen Endabschnitts 33 am Gelenkabschnitt 36 umsetzen lässt. Damit kann mit dem Shaver 30 der Figur 2 sowohl in Geradausrichtung, mit leichter Abwinkelung des distalen Endabschnitts 33 sowie mit einer deutlichen Abwinkelung, beispielsweise einer nahezu senkrechten Abwinkelung des Endabschnitts 33, gearbeitet werden, was dem Instrument einen erheblich vergrößerten Arbeitsbereich verschafft und auch schwierig zugängliche Arbeitspositionen zugänglich macht.

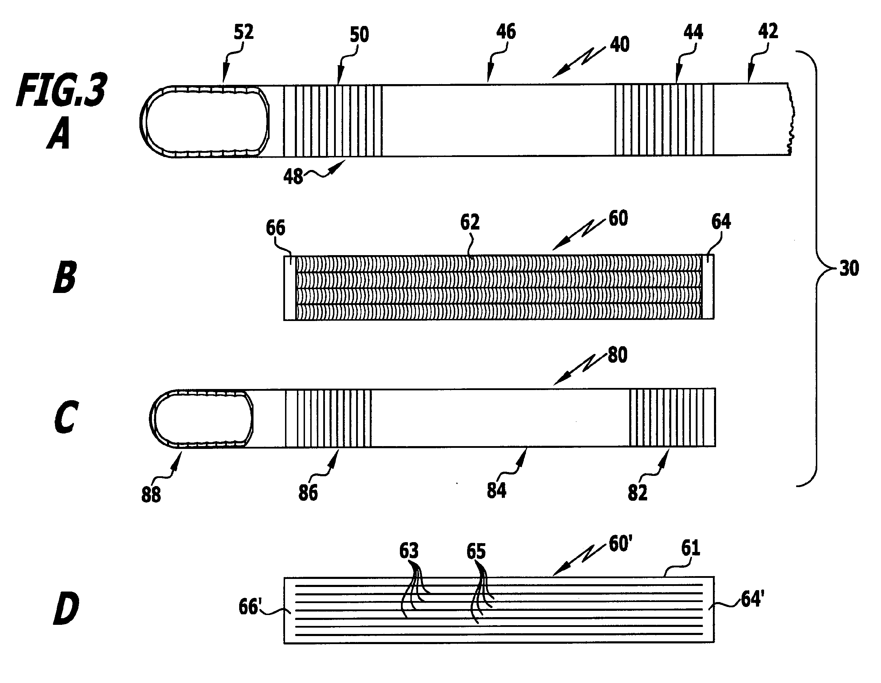

Der Aufbau des erfindungsgemäßen Shavers 30 wird anhand der Detailfiguren der Figuren 3A bis 3C im Einzelnen noch näher erläutert.

Figur 3A zeigt einen äußeren hohlzylindrischen Schaft 40 mit einem proximalen Endabschnitt, der einen Endbereich 42 und einen sich daran anschließenden proximalen flexiblen Abschnitt 44 umfaßt, einem sich weiterhin daran in Richtung zum distalen Ende 52 anschließenden biegesteifen Mittelabschnitt 46, auf den am distalen Endabschnitt 48 zunächst ein flexibler Abschnitt 50 folgt, an den dann eine Komponente eines Werkzeugs angeschlossen oder angeformt ist. Im vorliegenden Fall ist die Komponente des Werkzeugs an das distale Ende des äußeren Schafts 40 angeformt.

In diesen äußeren Schaft 40 wird dann ein in Figur 3B gezeigtes Steuerungselement 60 eingeschoben, welches eine Vielzahl, im vorliegenden Fall acht, parallel zur Längsrichtung des Instruments verlaufende Kraft übertragende Längselemente 62 aufweist, beispielsweise in Form von Kabeln oder Drähten.

Die Längselemente 62 sind an ihren proximalen und distalen Enden in Umfangsrichtung miteinander zu einem Ringbund 64, 66 verbunden. Die Länge des Steuerungselements 60 erstreckt sich, wie aus einem Vergleich der Darstellung in Figuren 3A und 3B ersichtlich, von dem proximalen Gelenkabschnitt 44 des äußeren Schafts 40 bis zum distalen Gelenkabschnitt 50 des äußeren Schafts 40.

Figur 3D zeigt eine alternative Ausführungsform eines Steuerungselements 60', welches aus einem einstückigen Röhrchen 61 beispielsweise durch Laserstrahlschneiden gefertigt ist.

Die in dem Röhrchen 61 durch Laserstrahlschneiden gebildeten Schlitze 63 verlaufen fast über die gesamte Länge des Röhrchens 61, so dass lediglich am proximalen und distalen Ende ungeschlitzte Ringbünde 64', 66' verbleiben, die die als Kraft übertragende Längselemente fungierenden Wandsegmente 65 jeweils miteinander verbinden.

In das Innere des hohlzylindrischen Steuerungselements 60 eingeschoben wird schließlich ein innerer hohlzylindrischer Schaft 80, wie er in Figur 3C dargestellt ist.

Auch der innere Schaft 80 umfasst am proximalen Ende einen Gelenkabschnitt oder flexiblen Abschnitt 82 sowie einen biegefesten Mittelabschnitt 84 und einen distalen Gelenkabschnitt oder flexiblen Abschnitt 86. An den distalen Gelenkabschnitt 86 angeschlossen ist eine Werkzeugkomponente 88, die, nachdem der innere Schaft 80 durch das Steuerungselement 60 hindurch in den äußeren Schaft 40 eingeschoben ist, in derselben Position wie die Werkzeugkomponente am distalen Ende 52 des äußeren Schafts 40 angeordnet ist.

Entsprechend einer bevorzugten Ausführungsform der Erfindung ist vorgesehen, dass der innere Schaft 80 gleichzeitig als Antriebselement fungiert, so dass bei einer Drehbewegung dann die Werkzeugkomponenten 88 und 52 zusammenwirken und beispielsweise in diesem Bereich in Kontakt kommende Gewebeteile über eine Schneid-, Abrasiv- oder Fräsfunktion entfernen können.

Nachdem der innere Schaft 80 ein freies Lumen aufweist, können solche Gewebeteile über das Lumen des inneren Schafts 80 nach außen zum proximalen Endabschnitt 42 gefördert und abgeführt werden.

Die Ausgestaltung der Gelenkabschnitte in Form der flexiblen Abschnitte 44, 50 bzw. 82, 86 des inneren bzw. äußeren Schafts kann vielfältig sein.

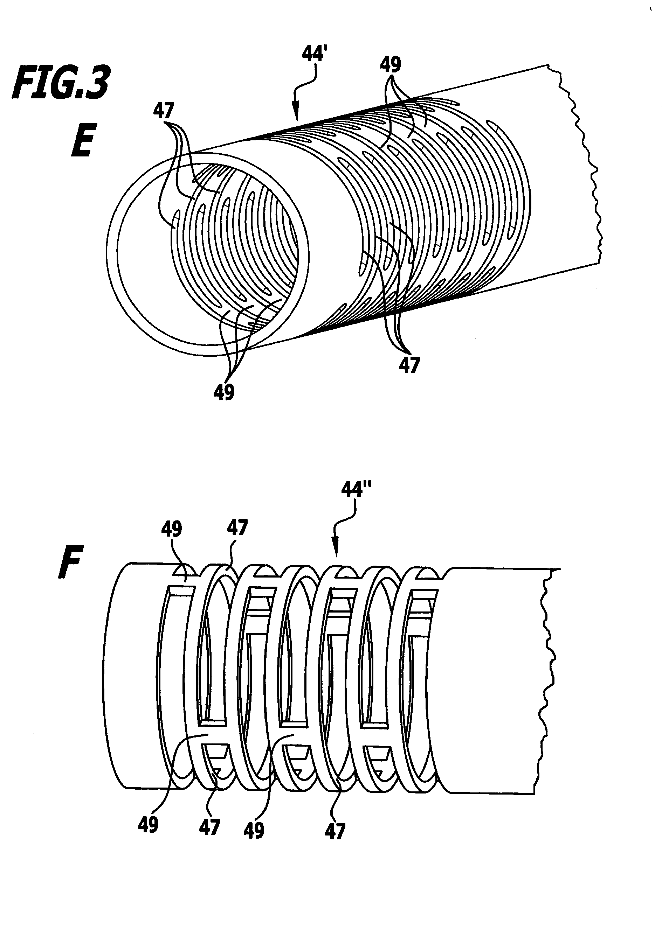

Die Figuren 3E und 3F zeigen zwei Varianten von verwandten Ausgestaltungen der flexiblen Abschnitte, hier in Form der Abschnitte 44' bzw. 44". Dieselbe Art der Ausgestaltung bietet sich auch für den flexiblen Abschnitt 50 an.

Gemeinsam ist den beiden Varianten die Verwendung einer Schlitzstruktur mit in Umfangsrichtung verlaufenden Schlitzen 47 in dem hohlzylindrischen Schaft. Vorzugsweise sind entlang einer Umfangslinie zwei oder mehr voneinander über Stege 49 getrennte Schlitze vorhanden. Da die Anordnung von Schlitzen entlang von nur einer Umfangslinie nur einen sehr kleinen Schwenkwinkel erlauben würde, sind bei typischen Schlitzstrukturen der Gelenkzone 44' eine Mehrzahl in Axialrichtung beabstandeter Umfangslinien mit Schlitzen 47 vorhanden. Bevorzugt sind in Axialrichtung benachbart angeordnete Schlitze 47 gegeneinander im Umfangsrichtung versetzt angeordnet, so dass sich Biegemöglichkeiten in mehreren Ebenen ergeben.

In Figur 3F sind zwei Schlitze 47 pro Umfangslinie vorhanden, die durch Stege 49 von einander getrennt sind. In Figur 3E sind es drei Schlitze 47. Die Schlitzstruktur umfasst in beiden Fällen typischerweise eine Vielzahl von Schlitzen 47, die entlang mehrerer gedachter und in Axialrichtung voneinander beabstandeter Umfangslinien angeordnet sind. Über die Wahl der Schlitzstruktur und die Anzahl der Schlitze lässt sich sehr einfach der zulässige Schwenkwinkel vorgeben und auch weitere Eigenschaften eines Gelenkabschnitts, wie z.B. die Biegefestigkeit, auf den jeweiligen Anwendungsfall anpassen.

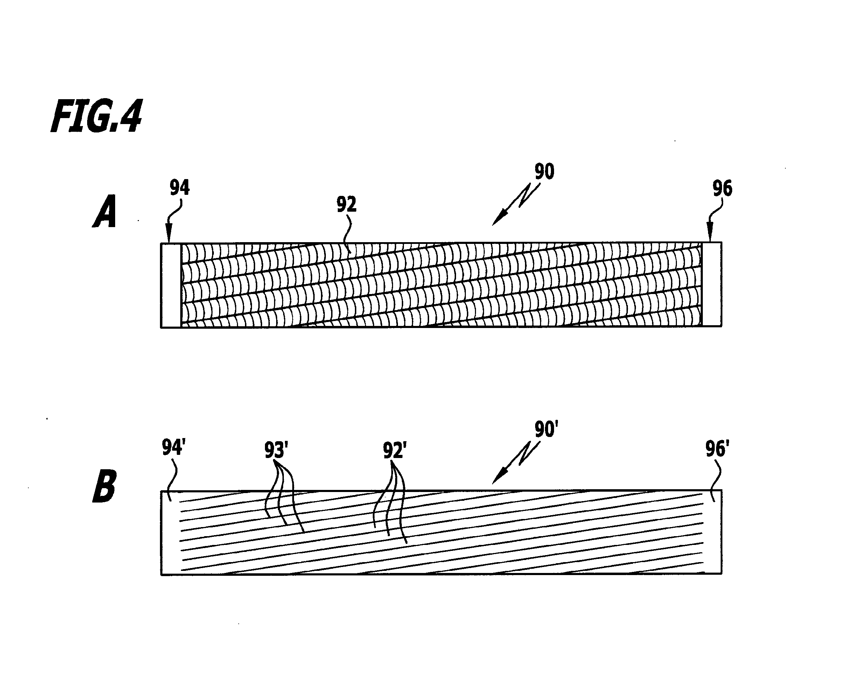

Figur 4A zeigt ein alternatives Steuerungselement 90, bei welchem die Kraft übertragenden Längselemente 92 mit ihren proximalen und distalen Enden an proximalen bzw. distalen Ringbünden 94, 96 angeschlossen sind. Im Gegensatz zu dem Steuerungselement 60, das in Figur 3B gezeigt ist, sind die Kraft übertragenden Längselemente 92 nicht geradlinig und parallel zur Längsachse des Steuerungselements 90 angeordnet, sondern entlang von Schraubenlinien, so dass die Enden der Längselemente 92 in Umfangsrichtung mit einem Winkelversatz an den Ringbünden 94, 96 enden. Der Winkelversatz in Umfangsrichtung beträgt bei dem in Figur 4A gezeigten Ausführungsbeispiel ca. 180°, mit der Folge, dass eine Schwenkbewegung des proximalen Endes des Instruments zu einer Schwenkbewegung des distalen Endabschnitts führt, die in derselben Schwenkebene jedoch in entgegengesetzter Richtung verläuft. Anstelle der in Figur 2 gezeigten S-Form wird dann eine U-förmig abgewinkelte Instrumentenkonfiguration erhalten.

Andere Winkeldifferenzen sind möglich; bei einem Winkelversatz von 90° erzielt man beispielsweise eine Schwenkbewegung des distalen Endabschnitts senkrecht zur Schwenkebene des proximalen Endabschnitts.

Figur 4B zeigt eine Variante eines Steuerungselements 90', welches ähnlich wie das Steuerungselement 60' der Figur 3D aus einem einstückigen Röhrchen durch Laserschneiden gebildet ist. Die dabei entstehenden Wandsegmente 92' sind durch Schlitze 93' von einander getrennt und nur im Bereich von Ringbünden 94', 96' kraftschlüssig miteinander verbunden. Die Vorteile des schraubenlinienförmigen Verlaufs der Wandsegmente sind dieselben wie bei dem Steuerungselement 90 mit dem schraubenlinienförmig verlaufenden Längselementen 92.

Die Ringsegmente 24 des Antriebselements 20 (Figur 1B) sind in Figur 5 im Einzelnen dargestellt und weisen einen ersten Endbereich 100 in einer ersten Axialrichtung auf und einen zweiten Endbereich 102 in der entgegengesetzten Axialrichtung. An seinem ersten Endbereich weist das Ringsegment 24 sechs Vorsprünge 104 auf, die regelmäßig in Umfangsrichtung verteilt an dem Ringsegment 24 angeordnet sind. An seinem axial entgegengesetzten Endbereich 102 weist das Ringsegment 24, korrespondierend zu den Vorsprüngen 104 geformte Ausnehmungen oder Rücksprünge 106 auf, wobei die Vorsprünge und Rücksprünge in Umfangsrichtung gesehen jeweils eine Trapezform aufweisen, wobei bei den Vorsprüngen 104 das in Axialrichtung freie Ende eine größere Ausdehnung in Umfangsrichtung aufweist als das am Ringsegment benachbart liegende Ende, während korrespondierend die Ausnehmungen 106 an ihrem inneren Ende eine größere Ausdehnung in Umfangsrichtung aufweisen als die in Richtung zum Endbereich 102 liegenden nach außen offenen Abschnitte. Im Fall der Ringsegmente 24, die in Figur 5 im Detail gezeigt sind, sind aufgrund der regelmäßigen Geometrie beide Endbereiche mit einer bis auf die spiegelbildliche Anordnung identischen Ausgestaltung versehen.

Dadurch, dass Vorsprünge 104 in korrespondierend geformte Rücksprünge 106 mit Spiel und trotzdem formschlüssig eingreifen, ist ein axialer Formschluss gegeben, so dass die Ringsegmente 24 in Axialrichtung miteinander verbunden und so zusammen in den äußeren Schaft 11 eingeführt bzw. aus diesem herausgezogen werden können.

Trotzdem bleibt aufgrund der bestehenden Spalte zwischen den Vorsprüngen 104 und den Rücksprüngen 106 ausreichend Spiel um eine gelenkige Verbindung benachbarter Ringsegmente 24 zu gewährleisten.

Die Vorsprünge 104 und die Ausnehmungen 106 weisen jeweils miteinander in Kontakt kommende Anlageflächen 108, 109 auf, die bei dem in Figur 5 gezeigten Ausführungsbeispiel großflächig eben ausgebildet sind. Damit ist eine optimale Drehmomentübertragung zwischen den einzelnen, aufeinander folgenden Ringsegmenten möglich.

Aufgrund der Vielzahl an Vor- und Rücksprüngen 104, 106 ergeben sich mehrere Schwenkachsen für die Verbindung zweier aufeinander folgender Ringsegmente, so dass das Antriebselement 20 dem gekrümmten Verlauf des hohlzylindrischen Schafts 11 folgen kann.

Die Vielzahl an ineinandergreifenden Vor- und Rücksprüngen 104, 106 mit trapezförmiger Konfiguration ist eine besonders sichere Verbindung in Axialrichtung gegeben.

Um das Antriebelement 20 mit seinem flexiblen Abschnitt und den diesen bildenden Ringsegmenten 24 insgesamt problemlos handhaben zu können, ist vorgesehen, dass die Ausdehnung der Vorsprünge 104 an ihrem freien Ende radial außen in Imfangsrichtung (a) größer ist als deren korrespondierende Ausdehnung auf der Innenseite (a').

Entsprechend sind die Ausnehmungen 106 an ihrem äußeren und inneren Umfang mit einer Breite b bzw. einer Breite b' dimensioniert, die auch in Radialrichtung einen Formschluss ermöglichen, so dass die Ringsegmente 24 unverlierbar miteinander verbunden und handhabbar sind.

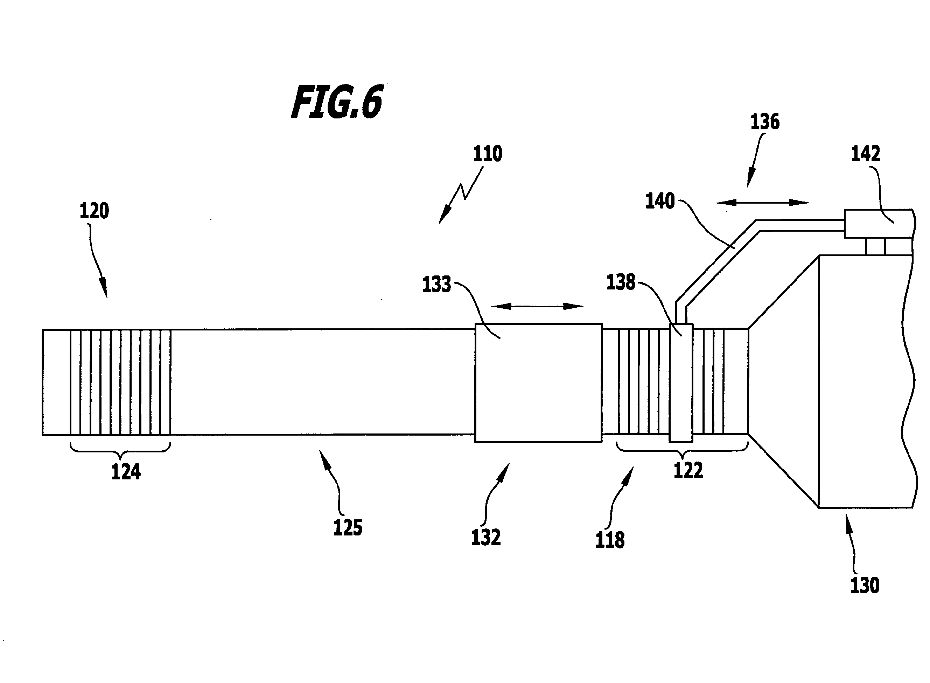

Figur 6 zeigt schließlich eine Variante der vorliegenden Erfindung in einer ersten Weiterbildung, bei der an ein Instrument 110 an dessen proximalen Endabschnitt 118 eine Handhabungsvorrichtung 130 angeschlossen ist.

Das Instrument 110 weist wie im Zusammenhang mit Figur 2 beschrieben proximale und distale Gelenkzonen 122 und 124 sind mit im Wesentlichen gleicher Länge ausgebildet, so dass sich bei einer Abwinklung des proximalen Endabschnittes 118 um z.B. 30° eine entsprechende Abwinklung des distalen Endabschnittes 120 ebenfalls um 30° ergibt. Die Richtung in der die Abwinklung des distalen Endabschnitts 120 erfolgt hängt von der Wahl des hier im Einzelnen nicht gezeigten Steuerungselements und der Festlegung der Enden der Kraft übertragenden Längselemente ab, wie dies weiter oben im Einzelnen beschreiben wurde.

Das in Figur 6 gezeigte Instrument 110 weist zusätzlich eine Haltevorrichtung 132 in Form einer Hülse 133 auf, die auf dem äußeren Schaft der Steuervorrichtung 110 mit dem Mittelabschnitt 125 überlappend längsverschieblich angeordnet ist.

Verschiebt man die Hülse 133 in Richtung zum proximalen Endabschnitt 118 und lässt die Hülse 133 mit dieser Gelenkzone 122 überlappen, so verkürzt sich die Gelenkzone 122, wodurch deren maximaler Biegewinkel beschränkt wird. Damit lässt sich variabel der zulässige Biegewinkel im Bereich des distalen Endabschnitts 120 einstellen, so dass zum Beispiel beim endoskopischen Abtragen von pathologischen Strukturen ein definierter Arbeitsbereich unter Sicht des Operateurs einstellbar ist.

Figur 6 enthält eine alternative Lösung zu der Haltevorrichtung 132 in Form der Haltevorrichtung 136, welche einen Ring 138 umfasst, der über einen zweifach gekröpften Steg 140 mit einer Geradführung 142 an der Handhabungsvorrichtung 130 längsverschieblich festgelegt ist. Über die Veränderung der Position des Ringes 138 entlang des Abschnittes 118 lässt sich, wie zuvor bezüglich der Hülse 133 erläutert, der für die Biegebewegung des proximalen Endabschnitts zur Verfügung stehende Teil der Gelenkzone 122 verkürzen, so dass wiederum nur ein eingeschränkter Biegewinkel auf Seiten des distalen Endabschnittes 120 zugelassen wird. Die Gelenkzonen können wie weiter oben beschrieben ausgebildet sein.

Darüber hinaus ist vorstellbar, dass sowohl im Falle der Hülse 133 als auch im Falle des Ringes 138 eine Feststellung in einer vorgegebenen Position, das heißt mit einer vorgegebenen Überlappung der Gelenkzone, vorgenommen werden kann, so dass der eingestellte beschränkte Arbeitsbereich auf Seiten des distalen Endabschnittes 120 gesichert ist.

Andererseits ist vorstellbar, die Hülse 133 auch in Richtung des distalen Gelenkabschnittes 120 zu verschieben, wobei dann bei einer entsprechenden Schwenkbewegung des proximalen Endabschnittes 118 eine übersetzte, das heißt stärkere, Schwenkbewegung im Bereich des distalen Endabschnittes 120 erfolgt.

Ebenso ist vorstellbar, Markierungen für die Position der Hülse 133 bzw. des Ringes 138 oder dessen Geradführung 142 vorzusehen, so dass eine einmal gefundene Winkelbeschränkung auch später immer wieder exakt eingestellt werden kann.

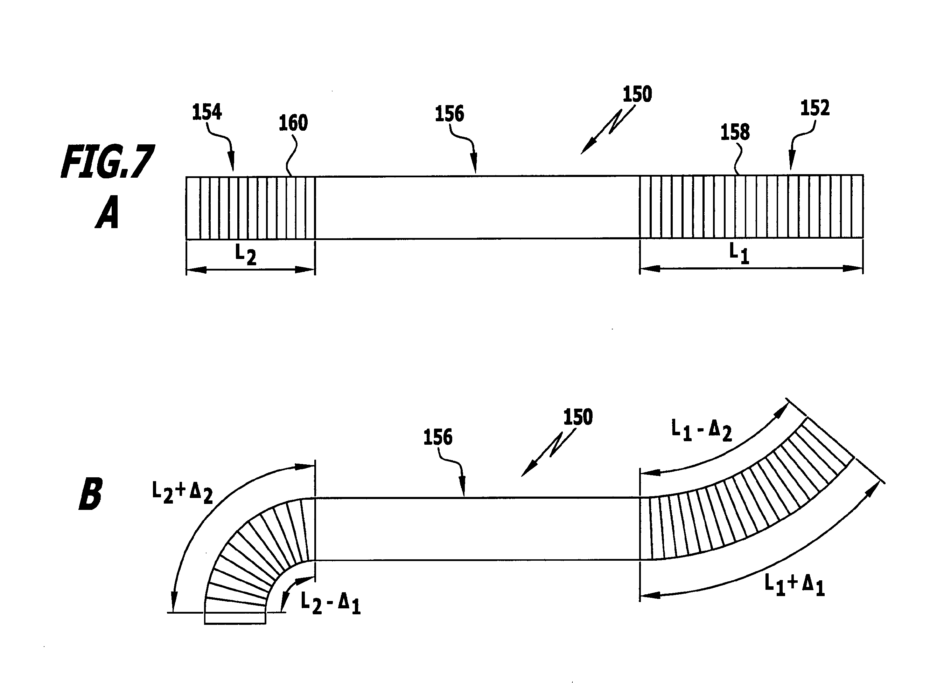

Zur Erläuterung des oben beschriebenen Effekts der Verstärkung der Schwenk- oder Biegebewegung am distalen Ende sei auf die Figur 7 verwiesen, die ein Instrument 150 zeigt, die einen proximalen Endabschnitt 152, einen distalen Endabschnitt 154 sowie einen dazwischen liegenden Mittelabschnitt 156 aufweist. Während der Mittelabschnitt 156 biegefest ausgebildet ist, beinhalten die proximalen und distalen Endabschnitte 152, 154 jeweils eine Gelenkzone 158 bzw. 160 mit einer in Axialrichtung gemessenen Länge L₁ bzw. L₂. Die Länge L₂ ist dabei kürzer gewählt als die Länge L₁. Die Figur 7A zeigt das Instrument 150 in der Grundstellung, in der keine Kräfte auf den proximalen Endabschnitt 152 wirken.

Wird der proximale Endbereich 152 aus der Axialrichtung herausgeschwenkt, wie dies in der Abbildung der Figur 7B verdeutlicht wird, so ergibt sich in der proximalen Gelenkzone 158 am Außenradius des gebogenen Endbereichs 152 eine vergrößerte Länge der Gelenkzone 158 von L₁ + Δ₁, am Innenradius ergibt sich eine verkürzte Länge von L₁ - Δ₂. Entsprechende Veränderungen in den Längen ergeben sich für den distalen Endabschnitt 154 mit einer Länge am Außenradius L₂ + Δ₂ und einer Länge am Innenradius von L₂ - Δ₁. Da die Längen L₁ und L₂ der Gelenkzonen 158, 160 unterschiedlich sind ergibt sich zwangsweise für den distalen Endabschnitt 154 eine verstärkte Biegebewegung um den von dem proximalen Endabschnitt vorgegebenen Längenänderungen folgen zu können.

Auch dieser Effekt lässt sich nutzen, um beispielsweise in einem proximal eingeschränkten Arbeitsbereich mit verhältnismäßig kleinen Schwenkbewegungen eine vollständige Nutzung des distal gegebenen Schwenkradius zu ermöglichen und distal einen möglichst großen Arbeitsbereich verfügbar zu machen.

Dieses Prinzip lässt sich mit der vorliegenden Erfindung variabel nutzen, indem über eine Haltevorrichtung (vgl. Figur 6) die Länge einer Gelenkzone im Verhältnis zur anderen variiert wird.

## Patentansprüche

1. Chirurgisches Instrument (10; 30) mit einem proximalen sowie einem distalen Endabschnitt (12, 16; 31, 33) und einem sich dazwischen erstreckenden Mittelabschnitt (14; 32), wobei das Instrument (10; 30) einen lang gestreckten, hohlen, sich vom proximalen zum distalen Endabschnitt des Instruments erstreckenden, äußeren Schaft (11; 40), ein in dem äußeren Schaft drehbar gelagertes Antriebselement (20; 80) sowie ein am distalen Endabschnitt des Instruments (10; 30) angeordnetes, mit dem Antriebselement gekoppeltes Bohr-, Schneid-, Abrasiv- oder Fräswerkzeug (22; 88) umfasst,
wobei das Antriebselement (20; 80) einen zwischen dem proximalen und distalen Endabschnitt (12, 16; 31, 33) angeordneten flexiblen Abschnitt (23) aufweist, der eine Mehrzahl an Ringsegmenten (24) umfasst, welche in Axialrichtung jeweils einen ersten und einen zweiten Endbereich (100; 102) aufweisen, wobei der erste Endbereich (100) zwei oder mehr in Axialrichtung abstehende Vorsprünge (104) und der zweite Endbereich zwei oder mehr die Vorsprünge aufnehmende Rücksprünge (106) umfasst und die Ringsegmente (24) über die Vor- und Rücksprünge gelenkig ineinander greifen,
**dadurch gekennzeichnet, dass** die Ringsegmente über die Vor- und Rücksprünge in Axial- und Radialrichtung miteinander formschlüssig verbunden sind,
dass der äußere Schaft (11) eine im Bereich des distalen Endabschnitts (16) des Instruments (10) angeordnete Gelenkzone (18) umfasst, die den distalen Endabschnitt (16) und den Mittelabschnitt (14) des Instruments (10) gelenkig miteinander verbindet, wobei der flexible Abschnitt (23) des Antriebselements (20) eine Länge in Axialrichtung aufweist, die zumindest im Wesentlichen der Länge der Gelenkzone (18) des äußeren Schafts (11) in Axialrichtung entspricht,
und dass das Instrument (10) ein Steuerungselement umfasst, mit welchem der distale Endabschnitt (16) des Instruments (10) gegenüber dem Mittelabschnitt (14) auslenkbar ist.

2. Instrument nach Anspruch 1, **dadurch gekennzeichnet, dass** die Zahl der Vorsprünge (104) und Rücksprünge (106) ungerade ist.

3. Instrument nach Anspruch 1 oder 2, **dadurch gekennzeichnet, dass** die Vorsprünge (104) und/oder die Rücksprünge (106) in Umfangsrichtung trapezförmig ausgebildet sind.

4. Instrument nach einem der Ansprüche 1 bis 3, **dadurch gekennzeichnet, dass** die Vorsprünge (104) und die Rücksprünge (106) in Umfangsrichtung miteinander in Kontakt tretende ebene Anlageflächen aufweisen.

5. Instrument nach einem der Ansprüche 1 bis 4, **dadurch gekennzeichnet, dass** der distale Endabschnitt (16) des Instruments (10) gegenüber dem Mittelabschnitt (14) reversibel auslenkbar ist.

6. Instrument nach einem der Ansprüche 1 bis 5, **dadurch gekennzeichnet, dass** das Steuerungselement (60) zwei oder mehr an Kraft übertragenden Längselementen (62) umfasst, welche in Umfangsrichtung des äußeren Schafts (40) regelmäßig verteilt angeordnet sind und sich vom proximalen Endabschnitt (31) bis zum distalen Endabschnitt (33) des Instruments (30) erstrecken, wobei optional der äußere Schaft (40) im proximalen Endabschnitt (31) eine zweite Gelenkzone umfasst und wobei die Kraft übertragenden Längselemente (62) an ihren jeweiligen proximalen und distalen Enden miteinander in Umfangsrichtung fest verbunden sind.

7. Instrument nach Anspruch 6, **dadurch gekennzeichnet, dass** die Kraft übertragenden Längselemente (62) von einander lateral beabstandet angeordnet sind.

8. Instrument nach Anspruch 6 oder 7, **dadurch gekennzeichnet, dass** die Kraft übertragenden Längselemente (62) in Umfangsrichtung gesehen in unterschiedlichen Winkelpositionen am proximalen und am distalen Endabschnitt enden und optional im Bereich des proximalen und/oder distalen Endabschnitts mit einer im Wesentlichen parallelen Ausrichtung zur Längsrichtung des Instruments angeordnet sind und/oder einen oder mehr Abschnitte aufweisen, welche schraubenlinienförmig zur Längsrichtung des Instruments angeordnet sind.

9. Instrument nach einem der Ansprüche 6 bis 8, **dadurch gekennzeichnet, dass** das Steuerungselement (60) torsionsfest ausgebildet ist.

10. Instrument nach einem der Ansprüche 1 bis 9, **dadurch gekennzeichnet, dass** die Gelenkzone(n) (35; 36) des äußeren Schafts (40) biegeelastisch ausgebildet ist (sind).

11. Instrument nach einem der Ansprüche 6 bis 10, **dadurch gekennzeichnet, dass** das Instrument (30) ferner einen inneren hohlzylindrischen Schaft (80) aufweist, welcher die Kraft übertragenden Längselemente (62) in Radialrichtung führt und eine Gelenkzone aufweist, die in Längsrichtung korrespondierend zur Position der Gelenkzone im Bereich des distalen Endabschnitts (33) des äußeren Schafts (40) angeordnet ist, wobei optional die Gelenkzone des inneren Schafts (80) im Wesentlichen dieselbe Länge aufweist wie die Gelenkzone des äußeren Schafts (40).

12. Instrument nach Anspruch 11, **dadurch gekennzeichnet, dass** der innere Schaft (80) innerhalb des Antriebselements angeordnet ist.

13. Instrument nach Anspruch 11, **dadurch gekennzeichnet, dass** der innere Schaft (80) zwischen dem Antriebselement und dem äußeren Schaft (40) angeordnet ist.

14. Instrument nach einem der Ansprüche 1 bis 13, **dadurch gekennzeichnet, dass** der äußere Schaft (40) eine zweite Gelenkzone aufweist, wobei eine der Gelenkzonen proximal und die andere Gelenkzone distal am Instrument (30) angeordnet ist, wobei optional die proximale Gelenkzone eine Ausdehnung in Längsrichtung des Instruments (30) aufweist, die von der Ausdehnung der distalen Gelenkzone verschieden ist und wobei ferner optional die Ausdehnung der proximalen und/oder distalen Gelenkzone des Instruments einstellbar ist, wobei vorzugsweise das Instrument eine Haltevorrichtung umfasst, mit der Teile einer Gelenkzone biegefest bezüglich der Längsrichtung des Instruments (30) oder einer sich an dessen proximalen oder distalen Endabschnitt anschließenden Funktionseinheit fixierbar sind.

15. Instrument nach einem der Ansprüche 1 bis 14, **dadurch gekennzeichnet, dass** das Antriebselement hohlzylindrisch ausgebildet ist und vorzugsweise den inneren Schaft (80) bildet.

## Claims

1. Surgical instrument (10; 30) having a proximal and a distal end section (12, 16; 31, 33) and a central section (14; 32) extending therebetween, the instrument (10; 30) comprising an elongated hollow outer shaft (11; 40) extending from the proximal to the distal end section of the instrument, a drive element (20; 80) rotatably mounted in the outer shaft and a boring, cutting, abrading or milling tool (22; 88) arranged at the distal end section of the instrument (10; 30) and coupled to the drive element,
the drive element (20; 80) comprising a flexible section (23) which is arranged between the proximal and the distal end section (12, 16; 31, 33) and comprises a plurality of ring segments (24) each of which has a first and a second end region (100; 102) in the axial direction, the first end region (100) comprising two or more projections (104) protruding in the axial direction and the second end region comprising two or more recesses (106) accommodating the projections, and the ring segments (24) intermeshing by means of the projections and recesses in an articulated manner,
**characterized in that** the ring segments are connected together in a positively locking manner in the axial and radial directions by the projections and recesses,
**in that** the outer shaft (11) comprises an articulation zone (18) which is arranged in the region of the distal end section (16) of the instrument (10) and connects the distal end section (16) and the central section (14) of the instrument (10) together in an articulated manner, a length of the flexible section (23) of the drive element (20) in the axial direction corresponding at least substantially to the length of the articulation zone (18) of the outer shaft (11) in the axial direction, and **in that** the instrument (10) comprises a control element with which the distal end section (16) of the instrument (10) is deflectable with respect to the central section (14).

2. Instrument in accordance with claim 1, **characterized in that** there is an odd number of projections (104) and recesses (106).

3. Instrument in accordance with claim 1 or 2, **characterized in that** the projections (104) and/or the recesses (106) have a trapezoidal shape in the circumferential direction.

4. Instrument in accordance with any one of claims 1 to 3, **characterized in that** the projections (104) and the recesses (106) have in the circumferential direction mutually contacting flat contact surfaces.

5. Instrument in accordance with any one of claims 1 to 4, **characterized in that** the distal end section (16) of the instrument (10) is deflectable with respect to the central section (14) in a reversible manner.

6. Instrument in accordance with any one of claims 1 to 5, **characterized in that** the control element (60) comprises two or more force transmitting longitudinal elements (62) which are arranged such that they are regularly distributed in the circumferential direction of the outer shaft (40) and extend from the proximal end section (31) to the distal end section (33) of the instrument (30), the outer shaft (40) optionally comprising a second articulation zone in the proximal end section (31), and the force transmitting longitudinal elements (62) being firmly connected together in the circumferential direction at their respective proximal and distal ends.

7. Instrument in accordance with claim 6, **characterized in that** the force transmitting longitudinal elements (62) are arranged in such a way that they are mutually laterally spaced.

8. Instrument in accordance with claim 6 or 7, **characterized in that**, as seen in the circumferential direction, the force transmitting longitudinal elements (62) end at different angular positions at the proximal and at the distal end section, and, optionally, in the region of the proximal and/or distal end section, are arranged in such a way that they are aligned substantially in parallel with the longitudinal direction of the instrument, and/or comprise one or more sections which are arranged helically with respect to the longitudinal direction of the instrument.

9. Instrument in accordance with any one of claims 6 to 8, **characterized in that** the control element (60) is formed so as to be torsion-resistant.

10. Instrument in accordance with any one of claims 1 to 9, **characterized in that** the articulation zone(s) (35; 36) of the outer shaft (40) is (are) bendable in a resilient manner.

11. Instrument in accordance with any one of claims 6 to 10, **characterized in that** the instrument (30) further comprises an inner hollow-cylindrical shaft (80) which guides the force transmitting longitudinal elements (62) in the radial direction and has an articulation zone which is arranged in the longitudinal direction in correspondence with the position of the articulation zone in the region of the distal end section (33) of the outer shaft (40), the articulation zone of the inner shaft (80) optionally being of substantially the same length as the articulation zone of the outer shaft (40).

12. Instrument in accordance with claim 11, **characterized in that** the inner shaft (80) is arranged within the drive element.

13. Instrument in accordance with claim 11, **characterized in that** the inner shaft (80) is arranged between the drive element and the outer shaft (40).

14. Instrument in accordance with any one of claims 1 to 13, **characterized in that** the outer shaft (40) has a second articulation zone, one of the articulation zones being arranged proximally and the other articulation zone distally on the instrument (30), the proximal articulation zone optionally having an extent in the longitudinal direction of the instrument (30), which is different from the extent of the distal articulation zone, and, furthermore, optionally, the extent of the proximal and/or distal articulation zone of the instrument being adjustable, the instrument preferably comprising a holding device with which parts of an articulation zone are fixable in a bending resistant manner with respect to the longitudinal direction of the instrument (30) or to a functional unit adjoining the proximal or distal end section thereof.

15. Instrument in accordance with any one of claims 1 to 14, **characterized in that** the drive element is in the form of a hollow cylinder and preferably forms the inner shaft (80).

## Revendications

1. Instrument chirurgical (10; 30) comportant un tronçon d'extrémité proximal ainsi que distal (12, 16; 31, 33) et, s'étendant entre ceux-ci, un tronçon central (14; 32),
l'instrument (10; 30) comportant un corps extérieur (11; 40) creux et allongé en longueur, qui s'étend du tronçon d'extrémité proximal à celui distal de l'instrument, un élément d'entraînement (20; 80) monté en rotation dans le corps extérieur, ainsi qu'un outil de perçage, de coupe, d'abrasion ou de fraisage (22; 88) agencé au niveau du tronçon d'extrémité distal de l'instrument (10; 30) et couplé à l'élément d'entraînement,
l'élément d'entraînement (20; 80) présentant un tronçon flexible (23), qui est agencé entre le tronçon d'extrémité proximal et distal (12, 16; 31, 33) et comprend une pluralité de segments annulaires (24) présentant chacun une première et une deuxième zone d'extrémité (100; 102),
la première zone d'extrémité (100) comportant deux protubérances (104) ou davantage, qui font saillie dans la direction axiale, et la deuxième zone d'extrémité deux retraits (106) ou davantage, qui accueillent lesdites protubérances, et les segments annulaires (24) s'emboitant mutuellement de manière articulée par l'intermédiaire des protubérances et des retraits, **caractérisé en ce que** les segments annulaires sont reliés les uns aux autres par complémentarité de formes dans la direction axiale et la direction radiale, par l'intermédiaire des protubérances et des retraits,
**en ce que** le corps extérieur (11) comprend une zone articulée (18), qui est agencée dans la zone du tronçon d'extrémité distal (16) de l'instrument (10), et relie de manière articulée le tronçon d'extrémité distal (16) et le tronçon central (14) de l'instrument (10), le tronçon flexible (23) de l'élément d'entraînement (20) présentant une longueur dans la direction axiale, qui correspond au moins sensiblement à la longueur de la zone articulée (18) du corps extérieur (11) dans la direction axiale,
et **en ce que** l'instrument (10) comporte un élément de commande à l'aide duquel le tronçon d'extrémité distal (16) de l'instrument (10) peut être dévié par rapport au tronçon central (14).

2. Instrument selon la revendication 1, **caractérisé en ce que** le nombre des protubérances (104) et des retraits (106) est impair.

3. Instrument selon la revendication 1 ou la revendication 2, **caractérisé en ce que** les protubérances (104) et/ou les retraits (106) sont d'une configuration trapézoïdale en direction périphérique.

4. Instrument selon l'une des revendications 1 à 3, **caractérisé en ce que** les protubérances (104) et les retraits (106) présentent des surfaces d'appui planes entrant mutuellement en contact dans la direction périphérique.

5. Instrument selon l'une des revendications 1 à 4, **caractérisé en ce que** le tronçon d'extrémité distal (16) de l'instrument (10) peut être dévié de manière réversible par rapport au tronçon central (14).

6. Instrument selon l'une des revendications 1 à 5, **caractérisé en ce que** l'élément de commande (60) comprend deux éléments longitudinaux (62) de transmission de force ou davantage, qui sont agencés de manière régulièrement répartie dans la direction périphérique du corps extérieur (40), et s'étendent du tronçon d'extrémité proximal (31) jusqu'au tronçon d'extrémité distal (33) de l'instrument (30), le corps extérieur (40) comportant de manière optionnelle une deuxième zone articulée dans le tronçon d'extrémité proximal (31), et les éléments longitudinaux (62) de transmission de force étant reliés mutuellement de manière fixe au niveau de leurs extrémités proximales et distales respectives.

7. Instrument selon la revendication 6, **caractérisé en ce que** les éléments longitudinaux (62) de transmission de force sont agencés de manière à être espacés latéralement les uns des autres.

8. Instrument selon la revendication 6 ou la revendication 7, **caractérisé en ce que** les éléments longitudinaux (62) de transmission de force se terminent, vus dans la direction périphérique, selon des positions angulaires différentes au niveau du tronçon d'extrémité proximal et du tronçon d'extrémité distal, et, optionnellement, sont agencés dans la zone du tronçon d'extrémité proximal et/ou distal, avec une orientation sensiblement parallèle à la direction longitudinale de l'instrument, et/ou présentent un ou plusieurs tronçons qui sont agencés sous forme de ligne en hélice par rapport à la direction longitudinale de l'instrument.

9. Instrument selon l'une des revendications 6 à 8, **caractérisé en ce que** l'élément de commande (60) est d'une configuration résistante en torsion.

10. Instrument selon l'une des revendications 1 à 9, **caractérisé en ce que** la ou les zone (s) articulée (s) (35; 36) du corps extérieur (40) présente(nt) une flexibilité élastique.

11. Instrument selon l'une des revendications 6 à 10, **caractérisé en ce que** l'instrument (30) comprend, par ailleurs, un corps intérieur (80) cylindrique creux, qui assure le guidage en direction radiale des éléments longitudinaux (62) de transmission de force, et présente une zone articulée agencée, dans la direction longitudinale, de manière à correspondre à la position de la zone articulée dans la région du tronçon d'extrémité distal (33) du corps extérieur (40), la zone articulée du corps intérieur (80) présentant optionnellement sensiblement la même longueur que la zone articulée du corps extérieur (40).

12. Instrument selon la revendication 11, **caractérisé en ce que** le corps intérieur (80) est agencé à l'intérieur de l'élément d'entraînement.

13. Instrument selon la revendication 11, **caractérisé en ce que** le corps intérieur (80) est agencé entre l'élément d'entraînement et le corps extérieur (40).

14. Instrument selon l'une des revendications 1 à 13, **caractérisé en ce que** le corps extérieur (40) présente une deuxième zone articulée, l'une des zones articulées est agencée de manière proximale et l'autre zone articulée de manière distale sur l'instrument (30), la zone articulée proximale présente, optionnellement, une étendue dans la direction longitudinale de l'instrument (30), qui est différente de l'étendue de la zone articulée distale, et l'étendue de la zone articulée proximale et/ou distale de l'instrument est par ailleurs, optionnellement, réglable, et l'instrument présente de préférence un dispositif de maintien, à l'aide duquel des parties d'une zone articulée peuvent être fixées de manière rigide en flexion par rapport à la direction longitudinale de l'instrument (30) ou à une unité fonctionnelle se raccordant à son tronçon d'extrémité proximal ou distal.

15. Instrument selon l'une des revendications 1 à 14, **caractérisé en ce que** l'élément d'entraînement est d'une configuration cylindrique creuse et forme de préférence le corps intérieur (80).
